# EUROPÄISCHE PATENTSCHRIFT

(11) **EP 2 714 755 B1**
(45) Veröffentlichungstag und Bekanntmachung des Hinweises auf die Patenterteilung: **26.04.2017**
(21) Anmeldenummer: 12725042.1
(22) Anmeldetag: 01.06.2012
(51) Int. Cl.: C08F 220/06, A61L 15/00, C08F 6/06

(54) **VERFAHREN ZUR KONTINUIERLICHEN HERSTELLUNG WASSERABSORBIERENDER POLYMERPARTIKEL**
METHOD FOR CONTINUOUS PRODUCTION OF WATER-ABSORBENT POLYMER PARTICLES
PROCÉDÉ DE PRÉPARATION CONTINUE DE PARTICULES POLYMÈRES HYDROABSORBANTES

(30) Priorität: 03.06.2011 EP 11168678
(43) Veröffentlichungstag der Anmeldung: 09.04.2014
(73) Patentinhaber: BASF SE, 67056 Ludwigshafen am Rhein (DE)
(72) Erfinder: FUNK, Rüdiger, 65527 Niedernhausen (DE); PFEIFFER, Thomas, 67459 Böhl-Iggelheim (DE); SCHRÖDER, Jürgen, 67071 Ludwigshafen (DE)
(74) Vertreter: BASF IP Association
(86) Internationale Anmeldenummer: PCT/EP2012/060428
(87) Internationale Veröffentlichungsnummer: WO 2012/164081

(56) Entgegenhaltungen:
- EP-A1- 1 840 137
- EP-A1- 2 396 029
- EP-B1- 2 178 926
- WO-A1-2004/003024
- WO-A1-2009/021921
- WO-A1-2010/090322
- WO-A1-2010/090324
- JP-K1- 2004 160 845
- JP-K1- 2004 352 899
- JP-K1- 2006 160 845
- US-A- 4 914 170
- US-A1- 2010 298 513
- US-A1- 2012 010 372

## Beschreibung

Die vorliegende Erfindung betrifft ein Verfahren zur kontinuierlichen Herstellung wasserabsorbierender Polymerpartikel, wobei die zur Herstellung der Polymerpartikel eingesetzte Acrylsäure eine geringe Reinheit aufweist.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49.2 g/cm² (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der wasserabsorbierenden Polymerpartikel kovalente Bindungen bilden können.

US 2010/0298513 offenbart ein Verfahren zur kontinuierlichen Herstellung wasserabsorbierender Teilchen durch Polymerisation eine wässrigen Lösung oder Suspension, die mindestens ein ethylenisch ungesättigtes Säuregruppen enthaltendes Monomer enthält, das gegebenenfalls teilweise neutralisiert ist, mindestens ein Vernetzungsmittel und mindestens einen Polymerisationsinitiator.

Aufgabe der vorliegenden Erfindung war die Bereitstellung eines verbesserten Verfahrens zur Herstellung wasserabsorbierender Polymerpartikel, insbesondere eines Verfahren bei dem auch stärker verunreinigte Monomere eingesetzt werden können.

Gelöst wurde die Aufgabe durch ein Verfahren zur kontinuierlichen Herstellung wasserabsorbierender Polymerpartikel, umfassend die Polymerisation einer Monomerlösung oder-suspension, enthaltend
a) Acrylsäure, wobei die Acrylsäure zumindest teilweise neutralisiert sein kann,
b) mindestens einen Vernetzer,
c) mindestens einen Initiator,
d) optional ein oder mehrere mit Acrylsäure copolymerisierbare ethylenisch ungesättigte Monomere und
e) optional ein oder mehrere wasserlösliche Polymere,
zu einem Polymergel, die Trocknung des erhaltenen Polymergels, die Zerkleinerung des getrockneten Polymergels zu Polymerpartikeln und die Klassierung der erhaltenen Polymerpartikel, dadurch gekennzeichnet, dass die Acrylsäure kontinuierlich zugeführt wird, die kontinuierlich zugeführte Acrylsäure eine Reinheit von 80 bis 99,5 Gew. % aufweist und sich die Reinheit der kontinuierlich zugeführten Acrylsäure in 24 Stunden um weniger als 0,5 Gew.-% ändert.

Die Reinheit der kontinuierlich zugeführten Acrylsäure beträgt von 80 bis 99,5 Gew.-%, bevorzugt von 90 bis 99 Gew.-%, besonders bevorzugt von 95 bis 98 Gew.-%. Die Änderung der Reinheit der kontinuierlich zugeführten Acrylsäure beträgt innerhalb von 24 Stunden weniger als 0,5 Gew.-%, bevorzugt weniger als 0,2 Gew.-%.

Die Reinheit der kontinuierlich zugeführten Acrylsäure ist 100 Gew.-% abzüglich der bei der Acrylsäureherstellung in der Acrylsäure verbleibenden Verunreinigungen und wird gaschromatographisch bestimmt. Dazu werden die detektierten Verunreinigungen von 100 Gew.-% abgezogen. Zu beachten ist hierbei, dass sich in der Acrylsäure vorhandenes Wasser nicht gaschromatographisch ermitteln lässt. Der Wassergehalt muss daher getrennt gemessen werden, beispielsweise durch Karl-Fischer-Titration, und muss ebenfalls mit abgezogen werden. Die Bestimmung der Reinheit von Acrylsäure mittels Gaschromatographie wird auch in der Monographie "Modern Superabsorbent Polymer Technology", F.L. Buchholz und A.T. Graham, Wiley-VCH, 1998, Seite 121, beschrieben.

Der vorliegenden Erfindung liegt die Erkenntnis zugrunde, dass sich der Einfluss einzelner Verunreinigungen bei der Herstellung wasserabsorbierender Polymerpartikel durch geeignete Maßnahmen kompensieren lässt. Um in einem kontinuierlichen Verfahren eine gleichbleibende Produktqualität zu erzielen, ist es daher lediglich notwendig die Konzentration der Verunreinigungen über die Zeit weitgehend konstant zu halten. Die Maßnahmen zur Kompensation der Verunreinigungen müssen daher während der kontinuierlichen Herstellung nicht ständig angepasst werden.

Das erfindungsgemäße Verfahren ermöglicht daher den Einsatz weniger aufwendig gereinigter Acrylsäure und somit eine deutlich kostengünstigere Herstellung wasserabsorbierende Polymerpartikel.

Die möglichen Verunreinigungen lassen sich grob unterteilen in regelnde, inhibierende, vernetzende und indifferente Verunreinigungen, wobei die Verunreinigungen anhand ihrer Wirkungen in Vorversuchen klassifiziert werden können.

Regelnde Verunreinigungen greifen in die Polymerisationskinetik ein, indem sie wachsende Polymerketten abbrechen und neue Polymerisationsketten starten. Dadurch steigen Zentrifugenretentionskapazität (CRC) und Extrahierbare an. Gleichzeitig sinkt die Absorption unter Druck (AUL0.3psi). Die Wirkung regelnder Verunreinigungen kann beispielsweise durch Erhöhung der Menge an Vernetzer b) kompensiert werden. Derartige Verunreinigungen können beispielsweise Acrolein, Allylalkohol, Isopropanol, Furan-2-aldehyd (2-Furfural) und Benzaldehyd sein.

Inhibierende Verunreinigungen verhindern bzw. verlangsamen die Polymerisation. Die Wirkung inhibierender Verunreinigungen kann beispielsweise durch Erhöhung der Menge an Initiator c) kompensiert werden. Derartige Verunreinigungen können beispielsweise Hydrochinon und Phenothiazin sein.

Vernetzende Verunreinigungen erhöhen den Vernetzungsgrad bei der Polymerisation. Dadurch sinken Zentrifugenretentionskapazität (CRC) und Extrahierbare. Gleichzeitig steigt die Absorption unter Druck (AUL0.3psi). Die Wirkung vernetzender Verunreinigungen kann beispielsweise durch Senkung der Menge an Vernetzer b) kompensiert werden. Derartige Verunreinigungen können beispielsweise Allylacrylat und Allylmethacrylat sein.

Indifferente Verunreinigungen haben keinen oder nur einen geringen Einfluss auf die Polymerisation. Derartige Verunreinigungen können beispielsweise Essigsäure und Propionsäure sein.

Das erfindungsgemäße Verfahren ermöglicht die Verwendung weniger aufwendig gereinigter Acrylsäure. Wichtig ist nur, dass sich die Konzentrationen der in der eingesetzten Acrylsäure vorhandenen Verunreinigungen über die Zeit wenig ändert. So ist es beispielsweise möglich Acrylsäure mittels einer kurzen Destillationskolonne zu reinigen und auf einfachem Weg eine Acrylsäure mit erhöhtem Gehalt an Allylacrylat und anderen Verunreinigungen zu erzeugen. Die Polymerisation könnte dann mit einfachen Vorversuchen an diese Acrylsäurequalität angepasst werden. Die Destillation selber könnte dann anhand einer Leitkomponente, wie Essigsäure, überwacht werden, d.h. die Destillation wird so betrieben, dass der Gehalt dieser Leitkomponente konstant gehalten wird.

In einer besonders bevorzugten Ausführungsform der vorliegenden Erfindung enthält die kontinuierlich zugeführte Acrylsäure
- mindestens 0,0005 Gew.-%, vorzugsweise mindestens 0,001 Gew.-%, besonders bevorzugt mindestens 0,002 Gew.-%, ganz besonders bevorzugt mindestens 0,0025 Gew.-%, einer regelnden Verunreinigung und/oder
- mindestens 0,00005 Gew.-%, vorzugsweise mindestens 0,0001 Gew.-%, besonders bevorzugt mindestens 0,0002 Gew.-%, ganz besonders bevorzugt mindestens 0,00025 Gew.-%, einer inhibierenden Verunreinigung und/oder
- mindestens 0,005 Gew.-%, vorzugsweise mindestens 0,01 Gew.-%, besonders bevorzugt mindestens 0,02 Gew.-%, ganz besonders bevorzugt mindestens 0,025 Gew.-%, einer vernetzenden Verunreinigung.

In einer ganz besonders bevorzugten Ausführungsform der vorliegenden Erfindung enthält die kontinuierlich zugeführte Acrylsäure
- von 0,0005 bis 0,1 Gew.-%, vorzugsweise von 0,001 bis 0,05 Gew.-%, besonders bevorzugt von 0,002 bis 0,02 Gew.-%, ganz besonders bevorzugt von 0,0025 bis 0,01 Gew.-%, einer regelnden Verunreinigung und/oder
- von 0,00005 bis 0,1 Gew.-%, vorzugsweise von 0,0001 bis 0,05 Gew.-%, besonders bevorzugt von 0,0002 bis 0,02 Gew.-%, ganz besonders bevorzugt von 0,00025 bis 0,01 Gew.-%, einer inhibierenden Verunreinigung und/oder
- von 0,005 bis 1 Gew.-% , vorzugsweise von 0,01 bis 0,5 Gew.-%, besonders bevorzugt von 0,02 bis 0,2 Gew.-%, ganz besonders bevorzugt von 0,025 bis 0,1 Gew.-%, einer vernetzenden Verunreinigung.

Die Verunreinigungen sind insbesondere Essigsäure, Acrolein, Ameisensäure, Formaldehyd, Propionsäure, Furan-2-aldehyd, Furan-3-aldehyd, Benzaldehyd, Protoanemonin, Maleinsäureanhydrid, Maleinsäure, Diacrylsäure, Allylacrylat, Benzoesäure, Hydrochinon und/oder Phenothiazin.

Die wasserabsorbierenden Polymerpartikel werden durch Polymerisation einer Monomerlösung oder -suspension hergestellt und sind üblicherweise wasserunlöslich.

Der Anteil an Acrylsäure und/oder deren Salzen an der Gesamtmenge der Monomeren beträgt vorzugsweise mindestens 50 mol-%, besonders bevorzugt mindestens 90 mol-%, ganz besonders bevorzugt mindestens 95 mol-%.

Die eingesetzte Acrylsäure enthält üblicherweise Polymerisationsinhibitoren, vorzugsweise Hydrochinonhalbether, als Lagerstabilisator.

Die Monomerlösung enthält daher vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf die unneutralisierte Acrylsäure. Beispielsweise kann zur Herstellung der Monomerlösung eine Acrylsäure mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen der Acrylsäure kovalente Bindungen ausbilden können. Weiterhin sind auch polyvalente Metallsalze, die mit mindestens zwei Säuregruppen der Acrylsäure koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,2 bis 0,6 Gew.-%, jeweils bezogen auf Acrylsäure. Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hy-droxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit Acrylsäure copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Weiteren mit Acrylsäure copolymerisierbare ethylenisch ungesättigte Monomere d) sind beispielsweise ethylenisch ungesättigte Carbonsäuren, wie Methacrylsäure und Itaconsäure, und ethylenisch ungesättigte Sulfonsäuren, wie Styrolsulfonsäure und 2-Acrylamido-2-methylpro-pansulfonsäure (AMPS).

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssiger Acrylsäure, beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 bis 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Feuchtegehalt gemäß der von der EDANA empfohlenen Testmethode Nr. WSP 230.2-05 "Mass Loss Upon Heating" bestimmt wird.

Die gemäß dem erfindungsgemäßen Verfahren hergestellten wasserabsorbierenden Polymerpartikel haben einen Anteil an Partikeln mit einer Partikelgröße von 300 bis 600 µm von vorzugsweise mindestens 30 Gew.-%, besonders bevorzugt mindesten 50 Gew.-%, ganz besonders bevorzugt mindestens 70 Gew.-%.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Die gemäß dem erfindungsgemäßen Verfahren hergestellten wasserabsorbierenden Polymerpartikel weisen eine Absorption unter einem Druck von 49,2 g/cm² von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² der wasserabsorbierenden Polymerpartikel beträgt üblicherweise weniger als 35 g/g. Die Absorption unter einem Druck von 49,2 g/cm² wird analog der von der EDANA empfohlenen Testmethode Nr. WSP 242.2-05 "Absorption Under Pressure, Gravimetric Determination" bestimmt, wobei statt eines Drucks von 21,0 g/cm² ein Druck von 49,2 g/cm² eingestellt wird.

### Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugene Plasky, 157, 1030 Brüssel, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

### Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

### Absorption unter einem Druck von 21,0 g/cm² (Absorption under Load)

Die Absorption unter einem Druck von 21,0 g/cm² (AUL0.3psi) der wasserabsorbierenden Polymerpartikel wird gemäß der von der EDANA empfohlenen Testmethode Nr. WSP 242.2-05 "Absorption Under Pressure, Gravimetric Determination" bestimmt.

### Absorption unter einem Druck von 49,2 g/cm² (Absorption under Load)

Die Absorption unter einem Druck von 49,2 g/cm² (AUL0.7psi) wird analog der von der EDANA empfohlenen Testmethode Nr. WSP 242.2-05 "Absorption Under Pressure, Gravimetric Determination" bestimmt, wobei statt eines Drucks von 21,0 g/cm² (AUL0.3psi) ein Druck von 49,2 g/cm² (AUL0.7psi) eingestellt wird.

### Extrahierbare

Der Gehalt an extrahierbaren Bestandteilen der wasserabsorbierenden Polymerpartikel wird gemäß der von der EDANA empfohlenen Testmethode Nr. WSP 270.2-05 "Extractable" bestimmt.

### Reinheit der Acrylsäure

Die Reinheitsbestimmung wird mittels eines Gaschromatographen mit Split-Injektor und Flammenionisationsdetektor durchgeführt. Es wird eine Kapillarsäure 30 m x 0,32 mm mit einer Filmstärke von 0,25 µm vom Typ J&W DB FFAB (Agilent Technologies, Waldbronn, Deutschland) verwendet. Die Injektortemperatur beträgt 180°C und die Detektortemperatur beträgt 240°C. Es wird folgendes Temperaturprogramm gewählt: 10 Minuten bei 120°C, anschließend mit 10°C/min bis 220°C und 15 Minuten bei 220°C. Das Split-Verhältnis beträgt 1 : 100 die Einspritzmenge 0,5 µl.

Temperaturprogramm, Splitverhältnis und Einspritzmenge können geringfügig variieren. Sie müssen so eingestellt werden, dass für alle Komponenten eine gute Trennung und ein hohes Signal-Rausch-Verhältnis erreicht wird.

Die Bestimmung wird mit 2-Ethylhexylacetat als interner Standard durchgeführt. Die detektierten Verunreinigungen werden mit dem Faktor 1 bewertet und zusammen mit dem in der Acrylsäure enthaltendem Wasser von 100 Gew.-% abgezogen. Wasser in Acrylsäure wird üblicherweise durch Karl-Fischer-Titration bestimmt.

### Beispiele

In den folgenden Versuchen wurde eine hochreine Acrylsäure eingesetzt. Die untersuchten Verunreinigungen waren in der eingesetzten Acrylsäure nicht nachweisbar.

### Beispiel 1

96 g Acrylsäure, 785 g wässrige Natriumacrylatlösung (37,3gew.-%ig), 115 g deionisiertes Wasser und 0,66 g 3-fach ethoxyliertes Glycerintriacrylat (ca. 85gew.-%ig) wurden für 30 Minuten durch Einleiten von Stickstoff vom Luftsauerstoff befreit. Die Polymerisation wurde in einem 2 Liter Kunststoffgefäß durch Zugabe von 2,34 g wässrige Natriumperoxodisulfatlösung (10,0gew.-%ig), 1,50 g wässrige Ascorbinsäurelösung (1,0gew.-%ig) und 1,50 g wässrige Wasserstoffperoxidlösung (1,0gew.-%ig) gestartet. Die maximale Polymerisationstemperatur (Tₘₐₓ) von ca. 108°C wurde nach 14 Minuten erreicht. 60 Minuten nach Erreichen von Tₘₐₓ wurde das Polymergel entnommen, mit einem Fleischwolf über eine 6 mm Lochscheibe zerkleinert, 60 Minuten bei 150°C in einem Umlufttrockenschrank getrocknet, dann mit einem Walzenstuhl nacheinander über die Spaltbreiten 1000µm, 600µm und 400µm gemahlen und durch Siebung des Mahlguts auf einen Partikelgrößenbereich von 150 bis 850 µm eingestellt. Die so erhaltenen wasserabsorbierenden Polymerpartikel hatten eine Zentrifugenretentionskapazität (CRC) von 40,3 g/g und eine Absorption unter Druck (AUL0.3psi) von 7,8 g/g.

### Beispiel 2

Es wurde verfahren wie unter Beispiel 1, jedoch wurde die Polymerisation in Gegenwart von 100 ppm Allylacrylat (bezogen auf Acrylsäure) durchgeführt. Die so erhaltenen wasserabsorbierenden Polymerpartikel hatten eine Zentrifugenretentionskapazität (CRC) von 37,6 g/g und eine Absorption unter Druck (AUL0.3psi) von 8,0 g/g.

### Beispiel 3

Es wurde verfahren wie unter Beispiel 1, jedoch wurde die Polymerisation in Gegenwart von 200 ppm Allylacrylat (bezogen auf Acrylsäure) durchgeführt. Die so erhaltenen wasserabsorbierenden Polymerpartikel hatten eine Zentrifugenretentionskapazität (CRC) von 36,3 g/g und eine Absorption unter Druck (AUL0.3psi) von 8,4 g/g.

**Tab. 1: Einfluss von Allylacrylat (vernetzende Verunreinigung)**

| | ethoxyliertes Glycerintriacrylat | Allylacrylat | CRC | AUL0.3psi |
|---|---|---|---|---|
| Bsp. 1 | 0,66 g | 0 ppm | 40,3 g/g | 7,8 g/g |
| Bsp. 2 | 0,66 g | 100 ppm | 37,6 g/g | 8,0 g/g |
| Bsp. 3 | 0,66 g | 200 ppm | 36,3 g/g | 8,4 g/g |

Die Tabelle zeigt, dass mit steigender Menge einer vernetzenden Verunreinigung die Zentrifugenretentionskapazität (CRC) fällt und die Absorption unter Druck (AUL0.3psi) steigt.

### Beispiel 4

Es wurde verfahren wie unter Beispiel 1, jedoch wurde die Polymerisation in Gegenwart von 100 ppm Allylacrylat (bezogen auf Acrylsäure) durchgeführt und die Menge an eingesetztem 3-fach ethoxylierten Glycerintriacrylat auf 0,55 g reduziert. Die so erhaltenen wasserabsorbierenden Polymerpartikel hatten eine Zentrifugenretentionskapazität (CRC) von 38,9 g/g und eine Absorption unter Druck (AUL0.3psi) von 8,0 g/g.

### Beispiel 5

Es wurde verfahren wie unter Beispiel 1, jedoch wurde die Polymerisation in Gegenwart von 100 ppm Allylacrylat (bezogen auf Acrylsäure) durchgeführt und die Menge an eingesetztem 3-fach ethoxylierten Glycerintriacrylat auf 0,44 g reduziert. Die so erhaltenen wasserabsorbierenden Polymerpartikel hatten eine Zentrifugenretentionskapazität (CRC) von 41,9 g/g und eine Absorption unter Druck (AUL0.3psi) von 7,9 g/g.

**Tab. 2: Kompensation einer vernetzenden Verunreinigung (Allylacrylat)**

| | ethoxyliertes Glycerintriacrylat | Allylacrylat | CRC | AUL0.3psi |
|---|---|---|---|---|
| Bsp. 2 | 0,66 g | 100 ppm | 37,6 g/g | 8,0 g/g |
| Bsp. 4 | 0,55 g | 100 ppm | 38,9 g/g | 8,0 g/g |
| Bsp. 5 | 0,44 g | 100 ppm | 41,9 g/g | 7,9 g/g |

Die Tabelle zeigt, dass sich der Einfluss der vernetztenden Verunreinigung durch Anpassung der Vernetzermenge kompensieren lässt.

### Beispiel 6

96 g Acrylsäure, 785 g wässrige Natriumacrylatlösung (37,3gew.-%ig), 115 g deionisiertes Wasser und 0,88 g 3-fach ethoxyliertes Glycerintriacrylat (ca. 85gew.-%ig) wurden für 30 Minuten durch Einleiten von Stickstoff vom Luftsauerstoff befreit. Die Polymerisation wurde in einem 2 Liter Kunststoffgefäß durch Zugabe von 0,78 g wässrige Natriumperoxodisulfatlösung (5,0gew.-%ig), 0,50 g wässrige Ascorbinsäurelösung (0,5gew.-%ig) und 0,50 g wässrige Wasserstoffperoxidlösung (0,5gew.-%ig) gestartet. Die maximale Polymerisationstemperatur (Tₘₐₓ) von ca. 106°C wurde nach 30 Minuten erreicht. 60 Minuten nach Erreichen von Tₘₐₓ wurde das Polymergel entnommen, mit einem Fleischwolf über eine 6 mm Lochscheibe zerkleinert, 60 Minuten bei 150°C in einem Umlufttrockenschrank getrocknet, dann mit einem Walzenstuhl nacheinander über die Spaltbreiten 1000µm, 600µm und 400µm gemahlen und durch Siebung des Mahlguts auf einen Partikelgrößenbereich von 150 bis 850 µm eingestellt. Die so erhaltenen wasserabsorbierenden Polymerpartikel hatten eine Zentrifugenretentionskapazität (CRC) von 29,2 g/g, eine Absorption unter Druck (AUL0.3psi) von 16,3 g/g und eine Absorption unter hohem Druck (AUL0.7psi) von 8,0 g/g.

### Beispiel 7

Es wurde verfahren wie unter Beispiel 6, jedoch wurde die Polymerisation in Gegenwart von 1 ppm Phenothiazin (bezogen auf Acrylsäure) durchgeführt. Nach 30 Minuten lag die Polymerisationstemperatur deutlich unter 100°C, erreichte auch nach weiteren 60 Minuten keine 100°C und konnte wegen eines unzureichenden Polymerisationsverlaufs nicht weiter aufgearbeitet werden.

Das Beispiel zeigt, dass sich die Monomerlösung in Gegenwart einer inhibierenden Verunreinigung nicht mehr polymerisieren lässt.

### Beispiel 8

Die in Gegenwart von 1 ppm Phenothiazin durchgeführte Polymerisation von (Beispiel 7) wurde wiederholt, jedoch mit erhöhter Menge an Polymerisationinitiatoren. Eingesetzt wurden 0,89 g wässrige Natriumperoxodisulfatlösung (5,0gew.-%ig), 0,57 g wässrige Ascorbinsäurelösung (0,5gew.-%ig) und 0,57 g wässrige Wasserstoffperoxidlösung (0,5gew.-%ig). Nach weiterer Aufarbeitung, wie in Beispiel 7 beschrieben, hatten die so erhaltenen wasserabsorbierenden Polymerpartikel eine Zentrifugenretentionskapazität (CRC) von 31,9 g/g, eine Absorption unter Druck (AUL0.3psi) von 17,7 g/g und eine Absorption unter hohem Druck (AUL0.7psi) von 7,8 g/g.

### Beispiel 9

Die in Gegenwart von 1 ppm Phenothiazin durchgeführte Polymerisation von (Beispiel 7) wurde wiederholt, jedoch mit erhöhter Menge an Polymerisationinitiatoren. Eingesetzt wurden 1,01 g wässrige Natriumperoxodisulfatlösung (5,0gew.-%ig), 0,65 g wässrige Ascorbinsäurelösung (0,5gew.-%ig) und 0,65 g wässrige Wasserstoffperoxidlösung (0,5gew.-%ig). Nach weiterer Aufarbeitung, wie in Beispiel 7 beschrieben, hatten die so erhaltenen wasserabsorbierenden Polymerpartikel eine Zentrifugenretentionskapazität (CRC) von 32,0 g/g, eine Absorption unter Druck (AUL0.3psi) von 13,1 g/g und eine Absorption unter hohem Druck (AUL0.7psi) von 7,6 g/g.

### Beispiel 10

Die in Gegenwart von 1 ppm Phenothiazin durchgeführte Polymerisation von (Beispiel 7) wurde wiederholt, jedoch mit erhöhter Menge an Polymerisationinitiatoren. Eingesetzt wurden 1,17 g wässrige Natriumperoxodisulfatlösung (10,0gew.-%ig), 0,75 g wässrige Ascorbinsäurelösung (1,0gew.-%ig) und 0,75 g wässrige Wasserstoffperoxidlösung (1,0gew.-%ig). 30 Minuten nach Initiierung der Polymerisation lag die Polymerisationstemperatur bei etwa 105°C und hatte mit etwa 105°C auch den Tₘₐₓ erreicht. Nach weiterer Aufarbeitung, wie in Beispiel 7 beschrieben, hatten die so erhaltenen wasserabsorbierenden Polymerpartikel eine Zentrifugenretentionskapazität (CRC) von 35,8 g/g, eine Absorption unter Druck (AUL0.3psi) von 9,3 g/g und eine Absorption unter hohem Druck (AUL0.7psi) von 7,5 g/g.

**Tab. 3: Kompensation einer inhibierenden Verunreinigung (Phenothiazin)**

| | Polymerisationsinitiator | Phenothiazin | CRC | AUL0.3psi | AUL0.7psi |
|---|---|---|---|---|---|
| Bsp. 6 | 100% | 0 ppm | 29,2 g/g | 16,3 g/g | 8,0 g/g |
| Bsp. 7 | 100% | 1 ppm | - *) | - *) | - *) |
| Bsp. 8 | 114% | 1 ppm | 31,9 g/g | 17,7 g/g | 7,8 g/g |
| Bsp. 9 | 130% | 1 ppm | 32,0 g/g | 13,1 g/g | 7,6 g/g |
| Bsp. 10 | 300% | 1 ppm | 35,8 g/g | 9,3 g/g | 7,5 g/g |

| | | | | | |
|---|---|---|---|---|---|
| *) unvollständige Polymerisation | | | | | |

Die Tabelle zeigt, dass sich der Einfluss der inhibierenden Verunreinigung durch Anpassung der Initiatormenge kompensieren lässt.

### Beispiel 11

96 g Acrylsäure , 785 g wässrige Natriumacrylatlösung (37,3gew.-%ig), 115 g deionisiertes Wasser und 0,88 g 3-fach ethoxyliertes Glycerintriacrylat (ca. 85gew.-%ig) wurde für 30 Minuten durch Einleiten von Stickstoff vom Luftsauerstoff befreit. Die Polymerisation wurde in einem 2 Liter Kunststoffgefäß durch Zugabe von 2,34 g wässrige Natriumperoxodisulfatlösung (2,0gew.-%ig), 2,40 g wässrige Ascorbinsäurelösung (0,2gew.-%ig) und 2,00 g wässrige Wasserstoffperoxidlösung (1,0gew.-%ig) gestartet. Es wurde eine maximale Polymerisationstemperatur (Tₘₐₓ) von ca. 110°C erreicht. 60 Minuten nach Erreichen von Tₘₐₓ wurde das Polymergel entnommen, mit einem Fleischwolf über eine 6 mm Lochscheibe zerkleinert, 60 Minuten bei 150°C in einem Umlufttrockenschrank getrocknet, dann mit einem Walzenstuhl nacheinander über die Spaltbreiten 1000µm, 600µm und 400µm gemahlen und durch Siebung des Mahlguts auf einen Partikelgrößenbereich von 150 bis 850 µm eingestellt. Die so erhaltenen wasserabsorbierenden Polymerpartikel hatten eine Zentrifugenretentionskapazität (CRC) von 35,4 g/g, eine Absorption unter Druck (AUL0.3psi) von 9,6 g/g und 13,9 Gew.-% Extrahierbare.

### Beispiel 12

Es wurde verfahren wie unter Beispiel 11, jedoch wurde die Polymerisation in Gegenwart von 10 ppm Allylalkohol (bezogen auf Acrylsäure) durchgeführt. Die so erhaltenen wasserabsorbierenden Polymerpartikel hatten eine Zentrifugenretentionskapazität (CRC) von 36,6 g/g, eine Absorption unter Druck (AUL0.3psi) von 8,5 g/g und 14,1 Gew.-% Extrahierbare.

### Beispiel 13

Es wurde verfahren wie unter Beispiel 11, jedoch wurde die Polymerisation in Gegenwart von 10 ppm Allylalkohol (bezogen auf Acrylsäure) durchgeführt und die Menge an eingesetztem 3-fach ethoxylierten Glycerintriacrylat auf 0,97 g erhöht. Die so erhaltenen wasserabsorbierenden Polymerpartikel hatten eine Zentrifugenretentionskapazität (CRC) von 34,1 g/g, eine Absorption unter Druck (AUL0.3psi) von 10,9 g/g und 11,5 Gew.-% Extrahierbare.

### Beispiel 14

Es wurde verfahren wie unter Beispiel 11, jedoch wurde die Polymerisation in Gegenwart von 10 ppm Allylalkohol (bezogen auf Acrylsäure) durchgeführt und die Menge an eingesetztem 3-fach ethoxylierten Glycerintriacrylat auf 1,06 g erhöht. Die so erhaltenen wasserabsorbierenden Polymerpartikel hatten eine Zentrifugenretentionskapazität (CRC) von 33,7 g/g, eine Absorption unter Druck (AUL0.3psi) von 12,0 g/g und 11,4 Gew.-% Extrahierbare.

**Tab. 4: Kompensation einer regelnden Verunreinigung (Allylalkohol)**

| | ethoxyliertes Glycerintriacrylat | Allylalkohol | CRC | AUL0.3psi | Extrahierbare |
|---|---|---|---|---|---|
| Bsp. 11 | 0,88 g | 0 ppm | 35,4 g/g | 9,6 g/g | 13,9 Gew.-% |
| Bsp. 12 | 0,88 g | 10 ppm | 36,6 g/g | 8,5 g/g | 14,1 Gew.-% |
| Bsp. 13 | 0,97 g | 10 ppm | 34,1 g/g | 10,9 g/g | 11,5 Gew.-% |
| Bsp. 14 | 1,06 g | 10 ppm | 33,7 g/g | 12,0 g/g | 11,4 Gew.-% |

Die Tabelle zeigt, dass sich der Einfluss der regelnden Verunreinigung durch Anpassung der Vernetzermenge kompensieren lässt.

### Beispiel 15

Es wurde verfahren wie unter Beispiel 11. Die so erhaltenen wasserabsorbierenden Polymerpartikel hatten eine Zentrifugenretentionskapazität (CRC) von 35,6 g/g, eine Absorption unter Druck (AUL0.3psi) von 9,2 g/g und 13,5 Gew.-% Extrahierbare.

### Beispiel 16

Es wurde verfahren wie unter Beispiel 11 jedoch wurde die Polymerisation in Gegenwart von 10ppm Acrolein (bezogen auf Acrylsäure) durchgeführt. Die so erhaltenen wasserabsorbierenden Polymerpartikel hatten eine Zentrifugenretentionskapazität (CRC) von 37,4 g/g, eine Absorption unter Druck (AUL0.3psi) von 8,4 g/g und 14,7 Gew.-% Extrahierbare.

### Beispiel 17

Es wurde verfahren wie unter Beispiel 11, jedoch wurde die Polymerisation in Gegenwart von 10 ppm Acrolein (bezogen auf Acrylsäure) durchgeführt und die Menge an eingesetztem 3-fach ethoxylierten Glycerintriacrylat auf 0,97 g erhöht. Die so erhaltenen wasserabsorbierenden Polymerpartikel hatten eine Zentrifugenretentionskapazität (CRC) von 35,5 g/g, eine Absorption unter Druck (AUL0.3psi) von 9,8 g/g und 12,1 Gew.-% Extrahierbare.

### Beispiel 18

Es wurde verfahren wie unter Beispiel 11 jedoch wurde die Polymerisation in Gegenwart von 10 ppm Acrolein (bezogen auf Acrylsäure) durchgeführt und die Menge an eingesetztem 3-fach ethoxylierten Glycerintriacrylat auf 1,06 g erhöht. Die so erhaltenen wasserabsorbierenden Polymerpartikel hatten eine Zentrifugenretentionskapazität (CRC) von 34,7 g/g, eine Absorption unter Druck (AUL0.3psi) von 10,3 g/g und 11,3 Gew.-% Extrahierbare.

**Tab. 5: Kompensation einer regelnden Verunreinigung (Acrolein)**

| | ethoxyliertes Glycerintriacrylat | Acrolein | CRC | AUL0.3psi | Extrahierbare |
|---|---|---|---|---|---|
| Bsp. 15 | 0,88 g | 0 ppm | 35,6 g/g | 9,2 g/g | 13,5 Gew.-% |
| Bsp. 16 | 0,88 g | 10 ppm | 37,4 g/g | 8,4 g/g | 14,7 Gew.-% |
| Bsp. 17 | 0,97 g | 10 ppm | 35,5 g/g | 9,8 g/g | 12,1 Gew.-% |
| Bsp. 18 | 1,06 g | 10 ppm | 34,7 g/g | 10,3 g/g | 11,3 Gew.-% |

Die Tabelle zeigt, dass sich der Einfluss der regelnden Verunreinigung durch Anpassung der Vernetzermenge kompensieren lässt.

### Beispiel 19

Es wurde verfahren wie unter Beispiel 11. Die so erhaltenen wasserabsorbierenden Polymerpartikel hatten eine Zentrifugenretentionskapazität (CRC) von 35,5 g/g, eine Absorption unter Druck (AUL0.3psi) von 9,5 g/g und 13,2 Gew.-% Extrahierbare.

### Beispiel 20

Es wurde verfahren wie unter Beispiel 11, jedoch wurde die Polymerisation in Gegenwart von 10 ppm 2-Furfural (bezogen auf Acrylsäure) durchgeführt. Die so erhaltenen wasserabsorbierenden Polymerpartikel hatten eine Zentrifugenretentionskapazität (CRC) von 36,2 g/g, eine Absorption unter Druck (AUL0.3psi) von 9,2 g/g und 14,0 Gew.-% Extrahierbare.

### Beispiel 21

Es wurde verfahren wie unter Beispiel 11, jedoch wurde die Polymerisation in Gegenwart von 10 ppm 2-Furfural (bezogen auf Acrylsäure) durchgeführt und die Menge an eingesetztem 3-fach ethoxylierten Glycerintriacrylat auf 0,97 g erhöht. Die so erhaltenen wasserabsorbierenden Polymerpartikel hatten eine Zentrifugenretentionskapazität (CRC) von 34,6 g/g, eine Absorption unter Druck (AUL0.3psi) von 10,4 g/g und 12,5 Gew.-% Extrahierbare.

### Beispiel 22

Es wurde verfahren wie unter Beispiel 11, jedoch wurde die Polymerisation in Gegenwart von 10 ppm 2-Furfural (bezogen auf Acrylsäure) durchgeführt und die Menge an eingesetztem 3-fach ethoxylierten Glycerintriacrylat auf 1,06 g erhöht. Die so erhaltenen wasserabsorbierenden Polymerpartikel hatten eine Zentrifugenretentionskapazität (CRC) von 34,0 g/g, eine Absorption unter Druck (AUL0.3psi) von 10,7 g/g und 12,0 Gew.-% Extrahierbare.

**Tab. 6: Kompensation einer regelnden Verunreinigung (2-Furfural)**

| | ethoxyliertes Glycerintriacrylat | 2-Furfural | CRC | AUL0.3psi | Extrahierbare |
|---|---|---|---|---|---|
| Bsp. 19 | 0,88 g | 0 ppm | 35,5 g/g | 9,5 g/g | 13,2 Gew.-% |
| Bsp. 20 | 0,88 g | 10 ppm | 36,2 g/g | 9,2 g/g | 14,0 Gew.-% |
| Bsp. 21 | 0,97 g | 10 ppm | 34,6 g/g | 10,4 g/g | 12,5 Gew.-% |
| Bsp. 22 | 1,06 g | 10 ppm | 34,0 g/g | 10,7 g/g | 12,0 Gew.-% |

Die Tabelle zeigt, dass sich der Einfluss der regelnden Verunreinigung durch Anpassung der Vernetzermenge kompensieren lässt.

## Patentansprüche

1. Verfahren zur kontinuierlichen Herstellung wasserabsorbierender Polymerpartikel, umfassend die Polymerisation einer Monomerlösung oder -suspension, enthaltend
a) Acrylsäure, wobei die Acrylsäure zumindest teilweise neutralisiert sein kann,
b) mindestens einen Vernetzer,
c) mindestens einen Initiator,
d) optional ein oder mehrere mit Acrylsäure copolymerisierbare ethylenisch ungesättigte Monomere und
e) optional ein oder mehrere wasserlösliche Polymere,
zu einem Polymergel, die Trocknung des erhaltenen Polymergels, die Zerkleinerung des getrockneten Polymergels zu Polymerpartikeln und die Klassierung der erhaltenen Polymerpartikel, **dadurch gekennzeichnet, dass** die Acrylsäure kontinuierlich zugeführt wird, die kontinuierlich zugeführte Acrylsäure eine Reinheit von 80 bis 99,5 Gew.-% aufweist und sich die Reinheit der kontinuierlich zugeführten Acrylsäure in 24 Stunden um weniger als 0,5 Gew.-% ändert.

2. Verfahren gemäß Anspruch 1, **dadurch gekennzeichnet, dass** die kontinuierlich zugeführte Acrylsäure eine Reinheit von 90 bis 99 Gew.-% aufweist.

3. Verfahren gemäß Anspruch 1 oder 2, **dadurch gekennzeichnet, dass** sich die Reinheit der kontinuierlich zugeführten Acrylsäure in 24 Stunden um weniger als 0,2 Gew.-% ändert.

4. Verfahren gemäß einem der Ansprüche 1 bis 3, **dadurch gekennzeichnet, dass** die kontinuierlich zugeführte Acrylsäure
- mindestens 0,0005 Gew.-% einer regelnden Verunreinigung und/oder
- mindestens 0,00005 Gew.-% einer inhibierenden Verunreinigung und/oder
- mindestens 0,005 Gew.-% einer vernetzenden Verunreinigung
enthält.

5. Verfahren gemäß einem der Ansprüche 1 bis 4, **dadurch gekennzeichnet, dass** die kontinuierlich zugeführte Acrylsäure
- von 0,001 bis 0,05 Gew.-% einer regelnden Verunreinigung und/oder
- von 0,0001 bis 0,05 Gew.-% einer inhibierenden Verunreinigung und/oder
- von 0,05 bis 0,5 Gew.-% einer vernetzenden Verunreinigung
enthält.

6. Verfahren gemäß einem der Ansprüche 1 bis 5, **dadurch gekennzeichnet, dass** die Acrylsäure a) zu 25 bis 95 mol-% neutralisiert ist.

7. Verfahren gemäß einem der Ansprüche 1 bis 3 und 6, **dadurch gekennzeichnet, dass** die Verunreinigung Essigsäure, Acrolein, Ameisensäure, Formaldehyd, Propionsäure, Furan-2-aldehyd, Furan-3-aldehyd, Benzaldehyd, Protoanemonin, Maleinsäureanhydrid, Maleinsäure, Diacrylsäure, Allylacrylat, Benzoesäure, Hydrochinonmonomethylether, Hydrochinon und/oder Phenothiazin ist.

8. Verfahren gemäß einem der Ansprüche 1 bis 7, **dadurch gekennzeichnet, dass** die wasserabsorbierenden Polymerpartikel einen Feuchtegehalt von höchstens 15 Gew.-% aufweisen.

9. Verfahren gemäß einem der Ansprüche 1 bis 8, **dadurch gekennzeichnet, dass** mindestens 95 Gew.-% der wasserabsorbierenden Polymerpartikel eine Partikelgröße von größer als 150 µm aufweisen.

10. Verfahren gemäß einem der Ansprüche 1 bis 9, **dadurch gekennzeichnet, dass** mindestens 95 Gew.-% der wasserabsorbierenden Polymerpartikel eine Partikelgröße von höchstens 850 µm aufweisen.

11. Verfahren gemäß einem der Ansprüche 1 bis 10, **dadurch gekennzeichnet, dass** die wasserabsorbierenden Polymerpartikel eine Zentrifugenretentionskapazität von mindestens 15 g/g aufweisen.

12. Verfahren gemäß einem der Ansprüche 1 bis 11, **dadurch gekennzeichnet, dass** die wasserabsorbierenden Polymerpartikel oberflächennachvernetzt sind.

## Claims

1. A process for continuously producing water-absorbing polymer particles, comprising the polymerization of a monomer solution or suspension comprising
a) acrylic acid which may be at least partly neutralized,
b) at least one crosslinker,
c) at least one initiator,
d) optionally one or more ethylenically unsaturated monomers copolymerizable with acrylic acid and
e) optionally one or more water-soluble polymers,
to give a polymer gel, the drying of the resulting polymer gel, the comminution of the dried polymer gel to give polymer particles and the classification of the resulting polymer particles, wherein the acrylic acid is supplied continuously, the acrylic acid supplied continuously has a purity of from 80 to 99.5% by weight and the purity of the acrylic acid supplied continuously varies by less than 0.5% by weight within 24 hours.

2. The process according to claim 1, wherein the acrylic acid supplied continuously has a purity of 90 to 99% by weight.

3. The process according to claim 1 or 2, wherein the purity of the acrylic acid supplied continuously varies by less than 0.2% by weight within 24 hours.

4. The process according to any of claims 1 to 3, wherein the acrylic acid supplied continuously comprises
- at least 0.0005% by weight of a regulating impurity, selected from acrolein, allyl alcohol, isopropanol, furan-2-aldehyde and benzaldehyde, and/or
- at least 0.00005% by weight of an inhibiting impurity, selected from hydroquinone and phenothiazine, and/or
- at least 0.005% by weight of a crosslinking impurity, selected from allyl acrylate and allyl methacrylate.

5. The process according to any of claims 1 to 4, wherein the acrylic acid supplied continuously comprises
- from 0.001 to 0.05% by weight of a regulating impurity, selected from acrolein, allyl alcohol, isopropanol, furan-2-aldehyde and benzaldehyde, and/or
- from 0.0001 to 0.05% by weight of an inhibiting impurity, selected from hydroquinone and phenothiazine, and/or
- from 0.05 to 0.5% by weight of a crosslinking impurity, selected from allyl acrylate and allyl methacrylate.

6. The process according to any of claims 1 to 5, wherein the acrylic acid a) has been neutralized to an extent of 25 to 95 mol%.

7. The process according to any of claims 1 to 6, wherein the impurity is water, acetic acid, acrolein, formic acid, formaldehyde, propionic acid, furan-2-aldehyde, furan-3-aldehyde, benzaldehyde, protoanemonin, maleic anhydride, maleic acid, diacrylic acid, allyl acrylate, benzoic acid, hydroquinone monomethyl ether, hydroquinone and/or phenothiazine.

8. The process according to any of claims 1 to 7, wherein the water-absorbing polymer particles have a moisture content of at most 15% by weight.

9. The process according to any of claims 1 to 8, wherein at least 95% by weight of the water-absorbing polymer particles have a particle size of greater than 150 µm.

10. The process according to any of claims 1 to 9, wherein at least 95% by weight of the water-absorbing polymer particles have a particle size not exceeding 850 µm.

11. The process according to any of claims 1 to 10, wherein the water-absorbing polymer particles have a centrifuge retention capacity of at least 15 g/g.

12. The process according to any of claims 1 to 11, wherein the water-absorbing polymer particles have been surface postcrosslinked.

## Revendications

1. Procédé pour la préparation continue de particules polymères absorbant l'eau, comprenant la polymérisation d'une solution ou d'une suspension de monomères, contenant
a) de l'acide acrylique, l'acide acrylique pouvant être neutralisé au moins partiellement,
b) au moins un réticulant,
c) au moins un initiateur,
d) éventuellement un ou plusieurs monomères éthyléniquement insaturés copolymérisables avec l'acide acrylique et
e) éventuellement un ou plusieurs polymères solubles dans l'eau,
en un gel polymère, le séchage du gel polymère obtenu, le broyage du gel polymère séché en particules polymères et la classification des particules polymères obtenues, **caractérisé en ce que** l'acide acrylique est alimenté en continu, l'acide acrylique alimenté en continu présente une pureté de 80 à 99,5% en poids et la pureté de l'acide acrylique alimenté en continu varie de moins de 0,5% en poids en 24 heures.

2. Procédé selon la revendication 1, **caractérisé en ce que** l'acide acrylique alimenté en continu présente une pureté de 90 à 99% en poids.

3. Procédé selon la revendication 1 ou 2, **caractérisé en ce que** la pureté de l'acide acrylique alimenté en continu varie de moins de 0,2% en 24 heures.

4. Procédé selon l'une quelconque des revendications 1 à 3, **caractérisé en ce que** l'acide acrylique alimenté en continu contient
- au moins 0,0005% en poids d'une impureté de régulation, choisie parmi l'acroléine, l'alcool allylique, l'isopropanol, le furan-2-aldéhyde et le benzaldéhyde et/ou
- au moins 0,00005% en poids d'une impureté inhibitrice, choisie parmi l'hydroquinone et la phénothiazine et/ou
- au moins 0,005% en poids d'une impureté de réticulation, choisie parmi l'acrylate d'allyle et le méthacrylate d'allyle.

5. Procédé selon l'une quelconque des revendications 1 à 4, **caractérisé en ce que** l'acide acrylique alimenté en continu contient
- de 0,001 à 0,05% en poids d'une impureté de régulation, choisie parmi l'acroléine, l'alcool allylique, l'isopropanol, le furan-2-aldéhyde et le benzaldéhyde et/ou
- de 0,0001 à 0,05% en poids d'une impureté inhibitrice, choisie parmi l'hydroquinone et la phénothiazine et/ou
- de 0,05 à 0,5% en poids d'une impureté de réticulation, choisie parmi l'acrylate d'allyle et le méthacrylate d'allyle.

6. Procédé selon l'une quelconque des revendications 1 à 5, **caractérisé en ce que** l'acide acrylique est neutralisé à raison de 25 à 95% en mole.

7. Procédé selon l'une quelconque des revendications 1 à 6, **caractérisé en ce que** l'impureté est l'eau, l'acide acétique, l'acroléine, l'acide formique, le formaldéhyde, l'acide propionique, le furan-2-aldéhyde, le furan-3-aldéhyde, le benzaldéhyde, la proto-anémonine, l'anhydride de l'acide maléique, l'acide maléique, l'acide diacrylique, l'acrylate d'allyle, l'acide benzoïque, l'hydroquinonemonométhyléther, l'hydroquinone et/ou la phénothiazine.

8. Procédé selon l'une quelconque des revendications 1 à 7, **caractérisé en ce que** les particules polymères absorbant l'eau présentent une teneur en humidité d'au plus 15% en poids.

9. Procédé selon l'une quelconque des revendications 1 à 8, **caractérisé en ce qu'**au moins 95% en poids des particules polymères absorbant l'eau présentent une grosseur de particule supérieure à 150 µm.

10. Procédé selon l'une quelconque des revendications 1 à 9, **caractérisé en ce qu'**au moins 95% en poids des particules polymères absorbant l'eau présentent une grosseur de particule d'au plus 850 µm.

11. Procédé selon l'une quelconque des revendications 1 à 10, **caractérisé en ce que** les particules polymères absorbant l'eau présentent une capacité de rétention dans une centrifugeuse d'au moins 15 g/g.

12. Procédé selon l'une quelconque des revendications 1 à 11, **caractérisé en ce que** les particules polymères absorbant l'eau sont postréticulées en surface.
